# FASCICULE DE BREVET EUROPEEN

(11) **EP 1 415 618 B1**
(45) Date de publication et mention de la délivrance du brevet: **31.01.2007**
(21) Numéro de dépôt: 03291979.7
(22) Date de dépôt: 07.08.2003
(51) Int. Cl.: A61F 2/06

(54) **Ensemble médical d'introduction d'une prothèse expansible dans un conduit, et son procédé de retenue**
Medizinische Anordnung zum Einführen einer dehnbahren Prothese in ein Gefäss und Verfahren zur Rückhaltung
Medical assembly for the insertion of an expandable prosthesis into a conduit and method for retaining

(30) Priorité: 09.08.2002 FR 0210160
(43) Date de publication de la demande: 06.05.2004
(73) Titulaire: B. Braun Medical SAS, 92100 Boulogne Billancourt (FR)
(72) Inventeur: Forber, Simon J. c/o B. Braun Medical SAS, 86360 Chasseneuil (FR); Masse, Sylvain c/o B. Braun Medical SAS, 86360 Chasseneuil (FR); Sandoz-Othenin, Damien c/o B. Braun Medical SAS, 86360 Chasseneuil (FR)
(74) Mandataire: Arnaud, Jean

(56) Documents cités:
- WO-A-01/17458
- WO-A-99/49812
- US-A- 6 120 522

## Description

L'invention concerne un ensemble médical d'introduction d'une prothèse expansible dans un conduit corporel, ainsi qu'un procédé de retenue d'une prothèse expansible dans une gaine.

L'invention s'applique aux prothèses expansibles de forme tubulaire qui peuvent être introduites dans des conduits corporels, tels que des vaisseaux, et on la décrit dans la suite dans le cas de l'introduction d'un stent ou élargisseur de vaisseau.

Au cours d'opérations chirurgicales, des chirurgiens introduisent des prothèses expansibles dans des parties spécifiques de conduits corporels. A cet effet, avant l'opération, une prothèse est disposée à l'état contracté près de l'extrémité d'une gaine, en général un cathéter. Pendant l'opération, un chirurgien introduit la gaine dans un vaisseau et, lorsque l'extrémité de la gaine occupe une position convenable, le chirurgien manoeuvre un poussoir pour chasser progressivement la prothèse de la gaine afin que, par son expansion, celle-ci vienne se plaquer au contact de la paroi interne du vaisseau.

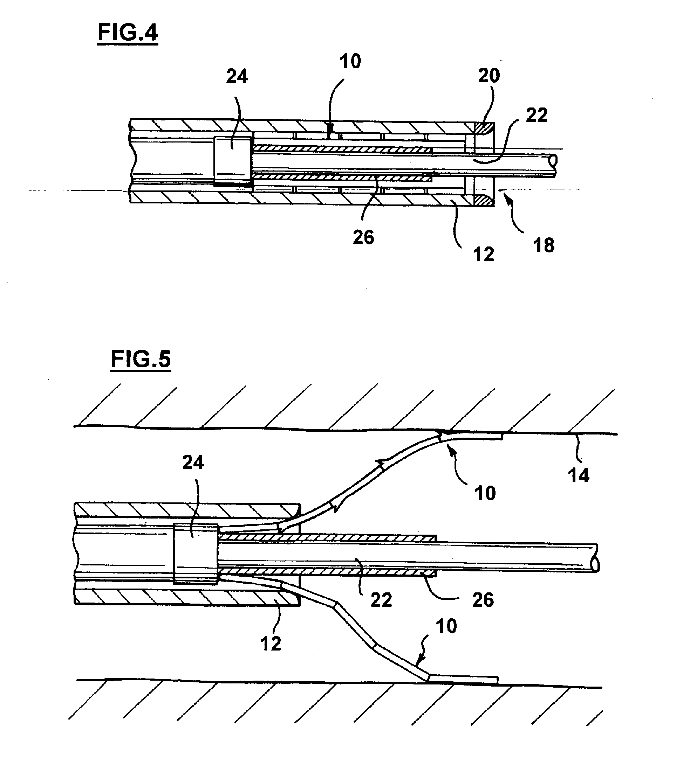

Cette situation est illustrée par l'examen rapide des figures 4 et 5 qui indiquent qu'une prothèse 10, disposée initialement dans une gaine 12, en est chassée progressivement afin que, par son expansion, elle vienne au contact de la paroi interne d'un conduit corporel 14.

On s'est rendu compte que ce procédé d'introduction d'une prothèse pouvait présenter un phénomène très défavorable qui est l'expulsion prématurée de la prothèse, même lorsque le chirurgien fait sortir la prothèse très progressivement de la gaine.

Plus précisément, surtout dans le cas d'une prothèse de faible longueur, par exemple de 20mm, placée dans un conduit dont le diamètre interne correspond à la partie supérieure de la plage de diamètres pour laquelle la prothèse est préconisée, celle-ci est expulsée à l'extrémité de la gaine et ne se positionne pas à l'emplacement voulu.

On décrit dans la suite, sans être limité par une théorie quelconque, une explication de ce phénomène d'expulsion.

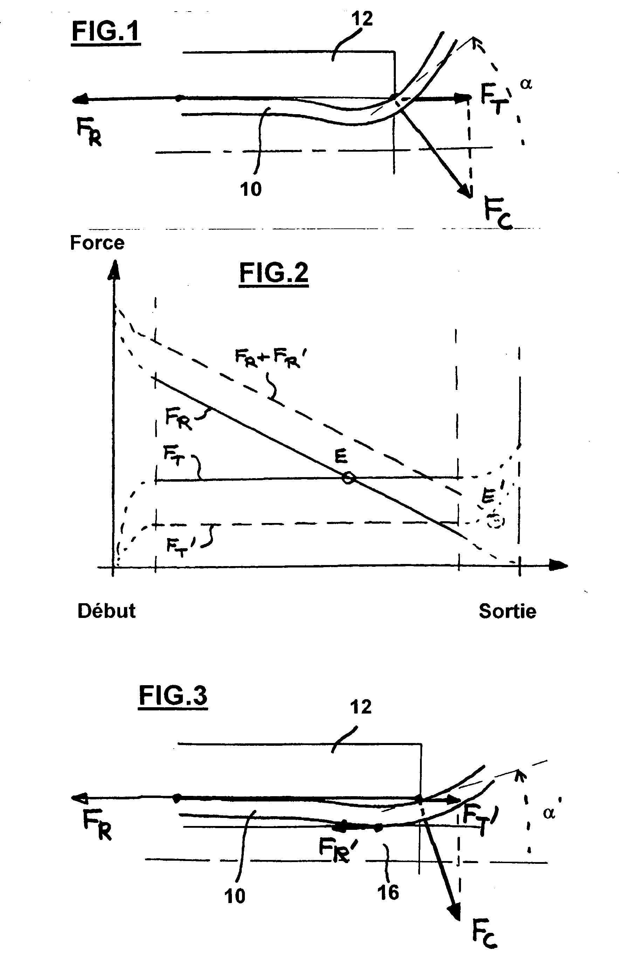

Lorsque la prothèse est entièrement placée à l'intérieur de la gaine (figure 4), elle s'applique contre la paroi interne de la gaine et n'a pas tendance à se déplacer le long de la gaine puisque sa force d'expansion l'applique contre la paroi interne de la gaine et crée une force de frottement (F_{T} sur la figure 1) qui est importante à la surface interne de la gaine, et résiste au déplacement de la prothèse.

Cependant, dès qu'une partie de la prothèse est sortie de l'extrémité de la gaine, elle a tendance à s'écarter radialement vers l'extérieur ; simultanément, comme la prothèse s'appuie au bord intérieur de l'orifice de la gaine, sa partie disposée immédiatement à l'intérieur de l'extrémité de la gaine s'écarte de la surface interne de la gaine sur une certaine distance, en se déformant radialement vers l'intérieur.

Au point de contact entre la prothèse et le bord interne de la gaine, la tangente à la prothèse forme un angle α. La force de réaction F_{C} appliquée par l'extrémité de la gaine à cause de la courbure de la prothèse (qui est élastique) est indiquée sur la figure 1 par la force F_{C}. Compte tenu de l'angle α, cette force de courbure de la prothèse F_{C} a une composante F_{T} en direction parallèle à l'axe de la gaine. Cette composante F_{T} est de sens opposé à la force de retenue F_{R} due au frottement de la partie de prothèse qui est au contact de la paroi interne de la gaine.

Lorsque la prothèse continue à sortir de la gaine, la surface de contact de la prothèse avec la paroi interne de la gaine, et donc la force de retenue F_{T}, diminuent progressivement. Au contraire, la composante F_{T} garde une valeur sensiblement constante : il arrive donc un moment où la force de retenue ou de frottement F_{R} devient inférieure à la composante F_{T} de la force de courbure de la prothèse 10. A partir de ce moment, la résultante des forces appliquées à la prothèse est dirigée vers la sortie, si bien que la prothèse est expulsée.

Si la prothèse est déjà venue au contact de la paroi du conduit corporel sur une certaine longueur au moment où se produit cette expulsion, la prothèse est cependant retenue et cette expulsion n'a pas lieu parce qu'une force antagoniste exercée par le conduit corporel par l'intermédiaire de la prothèse s'oppose à la résultante des forces précitées.

On peut mieux comprendre ce phénomène en référence au graphique de la figure 2.

Sur la figure 2, on a représenté par la courbe en trait plein F_{R} la variation de la force de retenue due au frottement de la prothèse à l'intérieur de la gaine. Cette force diminue linéairement entre le début de la poussée (position initiale du poussoir), c'est-à-dire lorsque la prothèse est encore entièrement à l'intérieur de la gaine, et la sortie de la gaine. A proximité de ces deux positions extrêmes, des phénomènes parasites peuvent se superposer, mais ils n'ont guère d'importance en réalité pour la compréhension du phénomène de l'expulsion.

On a aussi représenté sur la figure 2 la force de traction vers l'extérieur F_{T} qui est la composante de la force F_{C} due à la courbure de la prothèse. On a représenté cette force comme étant sensiblement constante, étant donné que la prothèse a le plus souvent une force d'expansion à peu près régulière sur toute sa longueur. On note que la force de traction vers l'extérieur F_{T} devient égale à la force de retenue F_{R} au point E. Si cet emplacement E est proche de la sortie de la gaine, le phénomène d'expulsion correspondant n'a pas grande importance en pratique puisque la prothèse est déjà bien retenue par le conduit corporel. Par contre, plus ce point E est proche du début de la poussée et plus l'expulsion de la prothèse est brutale.

Ce phénomène est très nuisible, car la prothèse vasculaire peut se trouver à un emplacement erroné, et peut se coincer. En outre, il n'est pas possible de la récupérer facilement à l'intérieur du corps humain. Il est donc très important d'empêcher ce phénomène.

On connaît déjà un certain nombre de systèmes destinés à résoudre ce problème de suppression de l'expulsion de la prothèse.

Le brevet des Etats-Unis d'Amérique n° 6 120 522 décrit un système dans lequel l'expulsion de la prothèse est retardée par disposition d'une membrane élastique à l'extrémité de la gaine. Cette membrane élastique est destinée à exercer des forces de frottement et de retenue qui retardent l'expulsion de la prothèse.

Le document WO 99/49 812 décrit un système dans lequel la partie la plus interne de la prothèse est retenue par des ailettes dépassant d'un poussoir spécial. Dans ce système, la prothèse ne peut se séparer du poussoir que lorsque celui-ci est sorti à l'extérieur de la gaine. Le poussoir a une forme spécifique à la prothèse, puisqu'il faut qu'il retienne celle-ci en avant et en arrière du poussoir.

Le document WO 01/17 458 décrit un système dans lequel le phénomène d'expulsion est empêché car, au moment où il doit se produire, un manchon vient coincer la prothèse entre lui-même et la gaine. La force de déplacement de la prothèse que doit exercer le chirurgien devient alors extrêmement importante, puisque la prothèse est coincée entre deux organes.

L'invention concerne une solution au problème précité qui diffère totalement des solutions des systèmes précités. On décrit cette solution du système selon l'invention dans son principe en référence à la figure 3.

Selon l'invention, deux phénomènes sont utilisés pour supprimer l'expulsion de la prothèse, le premier étant la réduction de la force qui a tendance à expulser la prothèse, et le second étant une retenue supplémentaire de la prothèse à l'intérieur de la gaine.

Plus précisément, comme représenté sur la figure 3, un organe interne désigné par la référence 16 fait subir une compression partielle radialement vers l'extérieur à la partie de la prothèse qui est courbée à l'intérieur de la gaine, très près de la sortie de celle-ci. Cet organe 16 empêche cette partie de la prothèse de se déformer autant que représenté sur la figure 1.

Plus précisément, cette compression partielle a deux effets : d'abord, elle réduit l'angle de la tangente à la prothèse à l'emplacement de contact avec le bord interne de la gaine 12, qui passe de l'angle α à l'angle α', et réduit donc la composante F_{T}' parallèle à l'axe de la gaine de la force de courbure F_{C}, et donc la force de traction vers l'extérieur de la prothèse. Ensuite, le contact de l'organe interne 16 qui exerce la compression partielle crée une force supplémentaire de retenue aux surfaces de contact, cette force pouvant être une force de frottement ou d'accrochage.

Plus précisément, comme l'indique la figure 2, d'une part la composante de traction vers l'extérieur F_{T}' est plus petite que la composante F_{T}, et d'autre part la force de retenue F_{R} due au frottement à l'intérieur de la gaine est accrue d'une force supplémentaire de retenue F_{R}'. On obtient donc une force totale de retenue F_{R} + F_{R}' .

On note facilement sur la figure 2 que l'emplacement E' auquel cette force de retenue accrue F_{R} + F_{R}' devient égale à la force réduite de traction F_{T}' est largement déplacé vers la sortie de la gaine, si bien que le phénomène d'expulsion n'est pratiquement plus observé.

Bien que l'exemple schématique de la figure corresponde au cas dans lequel la force de frottement F_{R}' de l'organe interne 16 est relativement faible, cette force peut être réglée par toutes sortes de moyens décrits dans la suite et peut être importante, afin qu'aucune expulsion ne se produise.

La solution selon l'invention est donc essentiellement une réduction de la force de traction de la prothèse par réduction de l'angle de sa tangente, et secondairement l'augmentation de la force de retenue.

Cette solution s'applique à un ensemble médical d'introduction d'une prothèse expansible dans un conduit corporel, et à un procédé de retenue d'une prothèse expansible dans une gaine.

Plus précisément, l'invention concerne un ensemble médical d'introduction d'une prothèse expansible dans un conduit corporel, du type qui comprend une gaine tubulaire ouverte à une extrémité distale, un élément allongé disposé pratiquement au centre de la gaine et laissant un espace intermédiaire en direction radiale par rapport à celle-ci, et un poussoir mobile dans la gaine tubulaire, la prothèse expansible étant introduite dans le conduit corporel par déplacement entre une première position de retenue dans laquelle elle est au moins pratiquement en appui contre la paroi interne de la gaine du fait de sa force d'expansion, et une seconde position expansée dans laquelle elle est en appui contre la paroi interne du conduit corporel, la prothèse expansible passant par des positions intermédiaires dans lesquelles une partie proximale de la prothèse est à l'intérieur de la gaine alors qu'une partie distale est à l'extérieur de la gaine, au-delà de l'extrémité distale de la gaine ; selon l'invention, la dimension radiale de l'espace intermédiaire entre la gaine et l'élément allongé est supérieure à la dimension radiale de la prothèse logée dans la gaine, afin que, quelque soient les tolérances sur les dimensions de la prothèse, de la gaine et de l'élément allongé, la prothèse ne soit pas au contact de l'élément allongé lorsqu'elle occupe sa première position, et la rigidité longitudinale de la prothèse et la dimension radiale de l'espace intermédiaire présentent une relation telle que la rigidité longitudinale de la prothèse est suffisamment élevée et la dimension radiale de l'espace intermédiaire est suffisamment petite pour que, en position intermédiaire dans laquelle une partie de la prothèse est au-delà de l'extrémité distale de la gaine, la partie de prothèse disposée dans la gaine à proximité de l'extrémité distale de celle-ci s'écarte de la gaine et vienne au contact de l'élément allongé.

De préférence, la surface extérieure de l'élément allongé comporte, au moins à un emplacement destiné à être en contact avec la surface interne de la prothèse, un organe créant une force de retenue de la prothèse en direction parallèle à l'axe de l'élément allongé.

Dans un mode de réalisation, l'organe créant une force de retenue est un organe qui crée une force de frottement, et il peut être choisi parmi un matériau élastomère, un matériau adhésif, un matériau tendre et un matériau rugueux.

Dans un autre mode de réalisation, l'organe créant une force de retenue est un organe créant une force d'accrochage, et celui-ci peut être choisi parmi des creux formés à la surface de l'organe allongé, tels qu'une série de gorges et un creux en hélice, et des saillies, telles qu'une série d'anneaux et une saillie en hélice. De préférence, les creux et les saillies ont des bords arrondis.

Dans un mode de réalisation avantageux, le poussoir mobile est solidaire de l'élément allongé.

L'invention concerne aussi un procédé de retenue d'une prothèse expansible dépassant partiellement de l'extrémité d'une gaine en s'écartant vers l'extérieur en direction radiale à l'extérieur de la gaine et vers l'intérieur en direction radiale juste à l'intérieur de l'extrémité de la gaine, la tangente à la prothèse à son emplacement de contact avec l'extrémité de la gaine formant un angle avec l'axe de la gaine ; selon l'invention, la procédé comprend la compression partielle, vers l'extérieur en direction radiale, de la partie qui s'écarte vers l'intérieur en direction radiale, provoquant une réduction de l'angle de la tangente à la prothèse avec l'axe de la gaine, à l'emplacement de contact avec l'extrémité de la gaine.

De préférence, la compression partielle s'accompagne de la création d'une force de retenue dans la région de contact de la partie qui subit la compression partielle et d'un organe de contact. La force de retenue peut être choisie parmi une force de frottement et une force de retenue mécanique par accrochage.

D'autres caractéristiques avantageuses de l'invention seront mieux comprises à la lecture de la description qui va suivre d'exemples de réalisation, faisant référence aux dessins annexés sur lesquels :
- la figure 1, déjà décrite, est un schéma utile pour la description du problème que résout l'invention ;
- la figure 2 est un graphique utile pour la compréhension de la mise en oeuvre de l'invention ;
- la figure 3 est un schéma analogue à celui de la figure 1 illustrant l'application de l'invention ;
- la figure 4 est une coupe d'un ensemble médical d'introduction selon un mode de réalisation de l'invention ;
- la figure 5 représente pratiquement l'ensemble de la figure 4 en position dans laquelle la prothèse est presque entièrement sortie de l'ensemble d'introduction ;
- les figures 6 et 7 représentent deux variantes de dispositifs mécaniques d'accrochage qui peuvent être utilisés selon l'invention ; et
- la figure 8 représente sous forme agrandie le détail cerclé de la figure 7.

La figure 4 représente un exemple d'ensemble médical d'introduction d'une prothèse dans un conduit corporel. La prothèse 10 sous forme contractée est disposée à l'intérieur d'une gaine 12 qui a orifice d'extrémité 18 délimité dans cet exemple par une bague 20 d'un matériau radio-opaque utile pour l'observation du déroulement d'une opération chirurgicale.

Un élément allongé 22 est placé au centre de la gaine et est solidaire d'un poussoir 24 qui est au contact de l'extrémité de la prothèse 10. L'élément allongé 22 est par exemple un cathéter. On note que l'élément allongé 22 porte à sa surface un revêtement 26. Dans l'exemple représenté, ce revêtement est constitué d'un élastomère de polyuréthanne. Cependant, il peut être constitué de toutes sortes de matériaux qui peuvent créer une force de retenue par frottement. Le matériau peut agir par son coefficient de frottement, son adhésivité, son élasticité, sa tendreté et/ou sa rugosité. Les hommes du métier peuvent facilement choisir un matériau convenable en fonction du résultat voulu, comme décrit dans la suite à propos de la réalisation de l'ensemble médical.

La figure 5 représente une phase de la mise en place qui est proche de sa fin. On note que la prothèse 10 a son extrémité distale qui est venue au contact de la paroi interne du conduit 14, alors que son extrémité proximale est encore placée à l'intérieur de la gaine, au contact du poussoir. Dans cette partie finale, on a dépassé les positions décrites en référence à la figure 3, car l'extrémité proximale de la prothèse s'est déjà séparée de la paroi interne de la gaine. La force de retenue ne comprend donc plus que la force de frottement sur le revêtement interne 26.

Sur les figures 4 et 5, on a représenté un revêtement 26 d'un matériau capable d'exercer une force de retenue par frottement. Il est aussi possible de créer une force de retenue par accrochage. En effet, les prothèses concernées sont le plus souvent formées de fils entrelacés délimitant des ouvertures et ayant donc de nombreux bords qui peuvent facilement s'accrocher à toute aspérité.

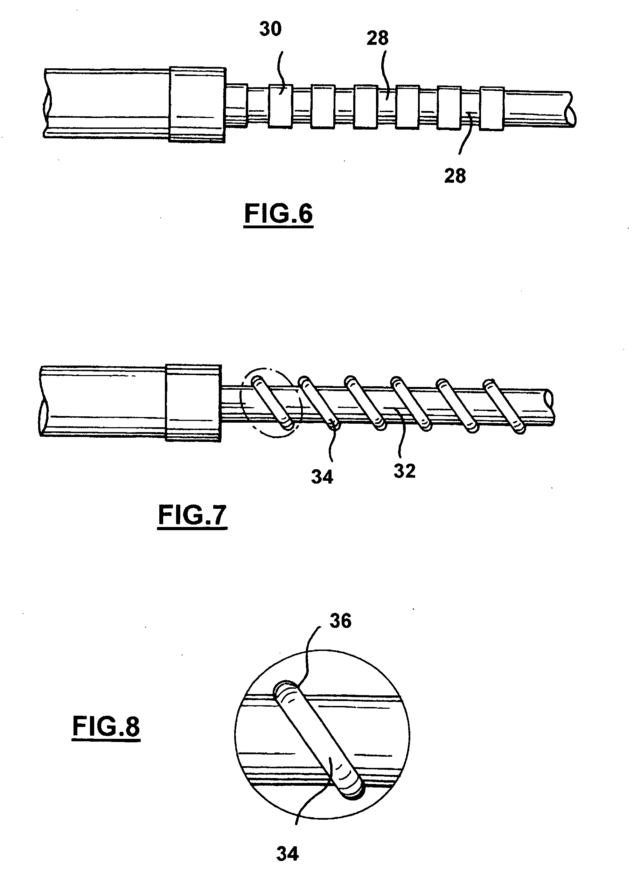

La figure 6 représente un premier exemple d'aspérités obtenues par formation de gorges 28 entre des saillies 30 de la surface extérieure de l'élément allongé.

La figure 7 représente un mode de réalisation dans lequel des saillies sont formées par une hélice disposée à la surface de l'organe allongé. Cette hélice 32 a des spires 34 qui ont avantageusement des bords arrondis 36, comme l'indique la figure 8. Les nombreux bords de la prothèse peuvent ainsi s'accrocher temporairement aux saillies formées par les spires 34 de l'hélice 32 ou les bords des saillies 30 placées entre les gorges 28.

La mise en oeuvre de l'invention nécessite le respect d'une relation entre la rigidité longitudinale de la prothèse et la dimension radiale de l'espace intermédiaire compris entre la gaine et l'élément allongé. Compte tenu des nombreux paramètres mis en oeuvre et de la très petite dimension des éléments utilisés (le diamètre externe du cathéter formant la gaine est le plus souvent de l'ordre de 2 à 2,5 mm), cette relation doit être déterminée de manière empirique. On décrit maintenant comment définir cette relation pour mettre en oeuvre l'invention.

Une fois définie la prothèse à mettre en place et la nature de la gaine, la prothèse est introduite partiellement dans la gaine afin qu'elle prenne la position indiquée sur la figure 1.

A ce moment, il est facile de déterminer les dimensions suivantes : le diamètre interne D1 de la gaine, le diamètre interne D2 de la prothèse à l'endroit où elle est au contact de la gaine, et le diamètre interne D3 de la prothèse à l'endroit où elle s'écarte le plus de la paroi intérieure de la gaine vers l'axe de la gaine.

On prend ainsi en compte les tolérances sur les dimensions de la prothèse, de la gaine et de l'élément allongé, si bien qu'il est facile de déterminer le diamètre externe maximal de l'élément allongé pour qu'il soit inférieur au diamètre D2, c'est-à-dire pour que l'organe allongé ne soit pas au contact de la prothèse lorsqu'elle occupe sa première position (contre la paroi intérieure de la gaine).

Pour que l'effet de l'invention soit obtenu, il faut que l'organe allongé ait un diamètre externe D supérieur au diamètre interne D3 de la prothèse à l'endroit où elle s'écarte le plus de la paroi intérieure de la gaine.

Le diamètre interne D de l'élément allongé 16 (cathéter muni éventuellement de son revêtement) est alors déterminé entre les deux diamètres précités D2 et D3. Plus le diamètre D se rapproche du diamètre D2, et plus l'effet de réduction de la force de traction est important. La force supplémentaire de retenue est essentiellement déterminée par la force de retenue créée par le contact des matériaux de la prothèse et de l'élément allongé (son revêtement ou ses creux ou saillies éventuellement).

Si la prothèse à une grande longueur, il peut être avantageux de ne pas augmenter excessivement la force de retenue, et on peut privilégier l'effet de réduction de la force de traction provoquant l'expulsion. Si la prothèse est courte, il est avantageux d'accroître la force de retenue en créant une force élevée de frottement et/ou d'accrochage à l'interface de la prothèse et de l'élément allongé.

Des essais réalisés avec des ensembles médicaux d'introduction selon l'invention ont montré qu'il était possible de supprimer totalement les à-coups de sortie des prothèses des gaines. On a aussi constaté qu'on n'observait pratiquement jamais de coincement de la prothèse. Celle-ci peut donc être introduite progressivement, en laissant la pleine maîtrise de la manoeuvre au chirurgien qui assure l'introduction.

## Revendications

1. Ensemble médical d'introduction d'une prothèse expansible dans un conduit corporel, du type qui comprend :
- une gaine tubulaire (12) ouverte à une extrémité distale,
- un élément allongé (22) disposé pratiquement au centre de la gaine tubulaire (12) et laissant un espace intermédiaire en direction radiale par rapport à celle-ci, et
- un poussoir (24) mobile dans la gaine tubulaire (12),
la prothèse expansible (10) étant introduite dans le conduit corporel par déplacement entre une première position de retenue dans laquelle elle est au moins pratiquement en appui contre la paroi interne de la gaine tubulaire (12) du fait de sa force d'expansion, et une seconde position expansée dans laquelle elle est en appui contre la paroi interne du conduit corporel,
la prothèse expansible (10) passant par des positions intermédiaires dans lesquelles une partie proximale de la prothèse (10) est à l'intérieur de la gaine tubulaire (12) alors qu'une partie distale est à l'extérieur de la gaine tubulaire (12), au-delà de l'extrémité distale de la gaine tubulaire (12),
**caractérisée en ce que**
la dimension radiale de l'espace intermédiaire entre la gaine tubulaire (12) et l'élément allongé (22) est supérieure à la dimension radiale de la prothèse (10) logée dans la gaine tubulaire (12), afin que, quelque soient les tolérances sur les dimensions de la prothèse (10), de la gaine tubulaire (12) et de l'élément allongé (22), la prothèse (10) ne soit pas au contact de l'élément allongé (22) lorsqu'elle occupe sa première position, et
la rigidité longitudinale de la prothèse (10) et la dimension radiale de l'espace intermédiaire présentent une relation telle que la rigidité longitudinale de la prothèse (10) est suffisamment élevée et la dimension radiale de l'espace intermédiaire est suffisamment petite pour que, en position intermédiaire dans laquelle une partie de la prothèse (10) est au-delà de l'extrémité distale de la gaine tubulaire (12), la partie de prothèse (10) disposée dans la gaine tubulaire (12) à proximité de l'extrémité distale de celle-ci s'écarte de la gaine tubulaire (12) et vienne au contact de l'élément allongé (22).

2. Ensemble selon la revendication 1, **caractérisé en ce que** la surface extérieure de l'élément allongé (22) comporte, au moins à un emplacement destiné à être en contact avec la surface interne de la prothèse (10), un organe créant une force de retenue de la prothèse (10) en direction parallèle à l'axe de l'élément allongé (22).

3. Ensemble selon la revendication 2, **caractérisé en ce que** l'organe créant une force de retenue est un organe (26) qui crée une force de frottement.

4. Ensemble selon la revendication 3, **caractérisé en ce que** l'organe (26) qui crée la force de frottement est choisi parmi un matériau élastomère, un matériau adhésif, un matériau tendre et un matériau rugueux.

5. Ensemble selon la revendication 2, **caractérisé en ce que** l'organe créant une force de retenue est un organe créant une force d'accrochage.

6. Ensemble selon la revendication 5, **caractérisé en ce que** l'organe créant une force d'accrochage comprend des creux formés à la surface de l'organe allongé, choisi parmi une série de gorges et un creux en hélice.

7. Ensemble selon la revendication 5, **caractérisé en ce que** l'organe créant une force d'accrochage comprend des saillies, choisies parmi une série d'anneaux et une saillie en hélice.

8. Procédé de retenue d'une prothèse expansible (10) dépassant partiellement de l'extrémité d'une gaine tubulaire (12) en s'écartant vers l'extérieur en direction radiale à l'extérieur de la gaine tubulaire (12) et vers l'intérieur en direction radiale juste à l'intérieur de l'extrémité de la gaine tubulaire (12), la tangente à la prothèse (10) à son emplacement de contact avec l'extrémité de la gaine tubulaire (12) formant un angle avec l'axe de la gaine tubulaire (12),
**caractérisé en ce qu**'il comprend la compression partielle, vers l'extérieur en direction radiale, de la partie qui s'écarte vers l'intérieur en direction radiale, provoquant une réduction de l'angle de la tangente à la prothèse (10) avec l'axe de la gaine tubulaire (12), à l'emplacement de contact avec l'extrémité de la gaine tubulaire (12).

9. Procédé selon la revendication 8, **caractérisé en ce que** la compression partielle s'accompagne de la création d'une force de retenue dans la région de contact de la partie qui subit la compression partielle et d'un organe de contact.

10. Procédé selon la revendication 9, **caractérisé en ce que** la force de retenue est choisie parmi une force de frottement et une force de retenue mécanique par accrochage.

## Claims

1. A medical assembly for the insertion of an expandable prosthesis into a body conduit, of the type which comprises:
- a tubular sheath (12) open at a distal end,
- an elongate member (22) disposed substantially in the centre of the tubular sheath (12) and leaving an intermediate space in a radial direction relative to the sheath, and
- a thruster (24) movable inside the tubular sheath (12),
the expandable prosthesis (10) being inserted into the body conduit by displacement between a first, retaining position in which it at least substantially bears against the inner wall of the tubular sheath (12) owing to its expansion force, and a second, expanded position in which it bears against the inner wall of the body conduit,
- the expandable prosthesis (10) passing through intermediate positions in which a proximal part of the prosthesis (10) is inside the tubular sheath (12), while a distal part is outside the tubular sheath (12), beyond the distal end of the tubular sheath (12),
**characterized in that**
the radial dimension of the intermediate space between the tubular sheath (12) and the elongate member (22) is greater than the radial dimension of the prosthesis (10) housed in the tubular sheath (12), so that, whatever the tolerances on the dimensions of the prosthesis (10), of the tubular sheath (12) and of the elongate member (22), the prosthesis (10) is not in contact with the elongate member (22) when the prosthesis assumes its first position, and
the longitudinal rigidity of the prosthesis (10) and the radial dimension of the intermediate space are in a relationship such that the longitudinal rigidity of the prosthesis (10) is sufficiently high and the radial dimension of the intermediate space is sufficiently small so that, in the intermediate position in which a part of the prosthesis (10) is beyond the distal end of the tubular sheath (12), the part of the prosthesis (10) disposed in the tubular sheath (12) in proximity to the distal end of the latter moves away from the tubular sheath (12) and comes into contact with the elongate member (22).

2. An assembly according to claim 1, **characterized in that** the outer surface of the elongate member (22) comprises, at least at a site intended to be in contact with the inner surface of the prosthesis (10), a device creating a force for retaining the prosthesis (10) in a direction parallel to the axis of the elongate member (22).

3. An assembly according to claim 2, **characterized in that** the device creating a retaining force is a device (26) which creates a frictional force.

4. An assembly according to claim 3, **characterized in that** the device (26) which creates the frictional force is selected from among an elastomeric material, an adhesive material, a soft material and a rough material.

5. An assembly according to claim 2, **characterized in that** the device creating a retaining force is a device creating an engagement force.

6. An assembly according to claim 5, **characterized in that** the device creating an engagement force comprises recesses formed at the surface of the elongate member, selected from among a series of grooves and a helical recess.

7. An assembly according to claim 5, **characterised in that** the device creating an engagement force comprises protuberances, selected from among a series of rings and a helical protuberance.

8. A method for retaining an expandable prosthesis (10) partially protruding from the end of a tubular sheath (12) while moving away towards the outside in a radial direction outside the tubular sheath (12) and towards the inside in a radial direction just inside the end of the tubular sheath (12), the tangent to the prosthesis (10) at its site of contact with the end of the tubular sheath (12) forming an angle with the axis of the tubular sheath (12),
**characterized in that** it comprises the partial compression, towards the outside in a radial direction, of the part which moves away towards the inside in a radial direction, bringing about a reduction in the angle of the tangent to the prosthesis (10) with the axis of the tubular sheath (12), at the site of contact with the end of the tubular sheath (12).

9. A method according to claim 8, **characterized in that** the partial compression is accompanied by the creation of a retaining force in the region of contact between the part which undergoes the partial compression and a contact member.

10. A method according to claim 9, **characterized in that** the retaining force is selected from among a frictional force and a force for mechanical retention by engagement.

## Patentansprüche

1. Medizinische Anordnung zum Einführen einer verlängerbaren Prothese in ein Körpergefäß, umfassend:
- eine röhrenförmige Hülse (12), die an einem distalen Ende offen ist,
- ein längliches Element (22), das praktisch in der Mitte der röhrenförmigen Hülse (12) angeordnet ist und einen Zwischenraum in radialer Richtung im Verhältnis zu dieser lässt, und
- eine Schubvorrichtung (24), die in der röhrenförmigen Hülse (12) beweglich ist,
wobei die verlängerbare Prothese (10) durch Verschieben zwischen einer ersten Rückhalteposition, in der sie aufgrund ihrer Ausdehnungskraft zumindest praktisch gegen die innere Wand der röhrenförmigen Hülse (12) anliegt, und einer zweiten ausgezogenen Position, in der sie gegen die innere Wand des Körpergefäßes anliegt, in das Körpergefäß eingeführt wird,
wobei die dehnbare Prothese (10) Zwischenpositionen durchläuft, in denen ein proximaler Teil der Prothese (10) sich innerhalb der röhrenförmigen Hülse (12) befindet, während ein distaler Teil sich außerhalb der röhrenförmigen Hülse (12) befindet, jenseits des distalen Endes der röhrenförmigen Hülse (12),
**dadurch gekennzeichnet, dass**
die radiale Abmessung des Zwischenraums zwischen der röhrenförmigen Hülse (12) und dem länglichen Element (22) größer ist als die radiale Abmessung der Prothese (10), die in der röhrenförmigen Hülse (12) aufgenommen ist, damit, unabhängig von den Abmessungstoleranzen der Prothese (10) der röhrenförmigen Hülse (12) und des länglichen Elements (22), die Prothese (10) sich nicht in Kontakt mit dem länglichen Element (22) befindet, wenn sie ihre erste Position einnimmt, und
die Längssteifigkeit der Prothese (10) und die radiale Abmessung des Zwischenraums ein solches Verhältnis aufweisen, dass die Längssteifigkeit der Prothese (10) groß genug ist und die radiale Abmessung des Zwischenraums klein genug ist, damit in der Zwischenposition, in der ein Teil der Prothese (10) sich jenseits des distalen Endes der röhrenförmigen Hülse (12) befindet, der Teil der Prothese (10), der in der röhrenförmigen Hülse (10) angeordnet ist, in der Nähe des distalen Endes dieser, sich von der röhrenförmigen Hülse (12) entfernt und in Kontakt mit dem länglichen Element (22) kommt.

2. Anordnung nach Anspruch 1, **dadurch gekennzeichnet, dass** die äußere Fläche des länglichen Elements (22) zumindest an einer Stelle, die dazu bestimmt ist, sich in Kontakt mit der inneren Fläche der Prothese (10) zu befinden, ein Mittel umfasst, das in paralleler Richtung zu der Achse des länglichen Elements (22) eine Rückhaltekraft der Prothese (10) erzeugt.

3. Anordnung nach Anspruch 2, **dadurch gekennzeichnet, dass** das Mittel, das eine Rückhaltekraft erzeugt, ein Mittel (26) ist, das eine Reibungskraft erzeugt.

4. Anordnung nach Anspruch 3, **dadurch gekennzeichnet, dass** das Mittel (26), das die Reibungskraft erzeugt, aus einem elastomeren Material, einem klebenden Material, einem weichen Material und einem rauen Material ausgewählt ist.

5. Anordnung nach Anspruch 2, **dadurch gekennzeichnet, dass** das Mittel, das eine Rückhaltekraft erzeugt, ein Mittel ist, das eine Haltekraft erzeugt.

6. Anordnung nach Anspruch 5, **dadurch gekennzeichnet, dass** das Mittel, das eine Haltekraft erzeugt, Ausnehmungen umfasst, die auf der Fläche des länglichen Mittels gebildet sind, das aus einer Reihe Rillen und einer Ausnehmung in Wendel form ausgewählt ist.

7. Anordnung nach Anspruch 5, **dadurch gekennzeichnet, dass** das Mittel, das eine Haltekraft erzeugt, Vorsprünge umfasst, die aus einer Reihe Ringe und einem Vorsprung in Wendelform ausgewählt sind,

8. Verfahren zur Rückhaltung einer verlängerbaren Prothese (10), die teilweise aus dem Ende einer röhrenförmigen Hülse (12) hervorsteht, indem sie sich nach außen in radialer Richtung außerhalb der röhrenförmigen Hülse (12) und nach innen in radialer Richtung entfernt, genau innerhalb des Endes der röhrenförmigen Hülse (12), wobei die Tangente der Prothese (10) an ihrer Kontaktstelle mit dem Ende der röhrenförmigen Hülse (12) einen Winkel mit der Achse der röhrenförmigen Hülse (12) bildet,
**dadurch gekennzeichnet, dass** es das teilweise Zusammendrücken nach außen in radialer Richtung des Teils umfasst, der sich nach innen in radialer Richtung entfernt, was eine Verringerung des Winkels der Tangente zur Prothese (10) mit der Achse der röhrenförmigen Hülse (12) bewirkt, an der Kontaktstelle mit dem Ende der röhrenförmigen Hülse (12).

9. Verfahren nach Anspruch 8, **dadurch gekennzeichnet, dass** das teilweise Zusammendrücken von der Erzeugung einer Rückhaltekraft im Kontaktbereich des Teils, der das teilweise Zusammendrücken erfährt, und eines Kontaktmittels begleitet wird.

10. Verfahren nach Anspruch 9, **dadurch gekennzeichnet, dass** die Rückhaltekraft aus einer Reibungskraft und einer mechanischen Rückhaltekraft durch Haften ausgewählt wird.
